(19) Europäisches Patentamt
European Patent Office
Office européen des brevets

(11) Publication number: **0 177 728 B1**

(12) **EUROPEAN PATENT SPECIFICATION**

(45) Date of publication of patent specification: **31.05.89**

(51) Int. Cl.[4]: **C 07 D 239/42,** A 61 K 31/505 // C07C129/12

(21) Application number: **85110654.2**

(22) Date of filing: **24.08.85**

(54) **Arphamenine derivatives, a process for their preparation and their use as medicament.**

(30) Priority: **03.09.84 JP 182788/84**

(43) Date of publication of application:
**16.04.86 Bulletin 86/16**

(45) Publication of the grant of the patent:
**31.05.89 Bulletin 89/22**

(84) Designated Contracting States:
**AT BE CH DE FR GB IT LI LU NL SE**

(56) References cited:
**EP-A-0 096 356**
**US-A-3 471 464**

**JOURNAL OF ANTIBIOTICS, vol. 36, no. 11, November 1983, pages 1576-1580; S. OHUCHI et al.: "The structure of arphamenines A and B"**
**CHEMISCHE BERICHTE, vol. 108, no. 3, 1975, pages 862-874; F-S. TJOENG et al.: "Vier Synthesewege zu (2-Pyrimidinylamino)-n-alkansäuren"**

**BIOCHEMISTRY, vol 14, no. 23, 1975, pages 5194-5199; H.F. GILBERT et al.: "Modification of arginine and lysine in proteins with 2,4-pentanedione"**

(73) Proprietor: **ZAIDAN HOJIN BISEIBUTSU KAGAKU KENKYU KAI**
**14-23, Kamiosaki 3-chome**
**Shinagawa-ku Tokyo (JP)**

(72) Inventor: **Umezawa, Hamao, Prof.**
**23, Toyotama-kita 4-chome**
**Nerima-ku Tokyo (JP)**
Inventor: **Takeuchi, Tomio**
**1-11, Higashigotanda 5-chome**
**Shinagawa-ku Tokyo (JP)**
Inventor: **Aoyagi, Takaaki**
**3-6, Honkugenuma 3-chome**
**Fujisawa-city Kanagawa Prefecture (JP)**
Inventor: **Ohuchi, Shohkichi**
**2-194, Todehoncho Saiwa-ku**
**Kawasaki-city Kanagawa Prefecture (JP)**

(74) Representative: **Becker, Heinrich Karl Engelbert, Dr. et al**
**HOECHST AKTIENGESELLSCHAFT Central Patent Department P.O. Box 80 03 20**
**D-6230 Frankfurt am Main 80 (DE)**

**EP 0 177 728 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

(56) References cited:

**ARCHIV DER PHARMAZIE, vol. 311, no. 4, 1978, pages 351-360, Weinheim; A. KREUTZBERGER et al.: "Analgetika, 5. Mitt. 2-(Äthylamino)-pyrimidine"**

**JOURNAL OF ANTIBIOTICS, vol. 37, no. 12, December 1984, pages 1741-1743; S. OHUCHI et al.: "Structure-activity relationships among derivatives of arphamenines, inhibitors of aminopeptidase B"**

## Description

This invention relates to new arphamenine-related compounds and pharmaceutically acceptable salts thereof which exhibit immunopotentiating activity, and this invention also relates to uses of these compounds.

The aminopeptidase-inhibitory activity as well as anti-tumor or immunopotentiating activity, of arphamenines has already been described (Japanese patent application first publication "KOKAI" No. 212791/83).

The chemical structure of an arphamenine is represented by the following general formula:

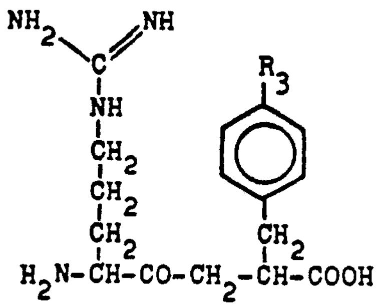

wherein $R_3$ is a hydrogen atom or a hydroxyl group. Herein, arphamenine is a generic name of arphamenines A and B. The compound of the general formula above wherein R is a hydrogen atom denotes the arphamenine A, and the compound of the general formula where R is a hydroxyl group denotes the arphamenine B. The structure determination of arphamenines A and B via their dimethylpyrimidine derivatives is reported in J. Antibiotics *36* (1983), p. 1576—1580, especially p. 1576.

It has been also found that benzylmalic acid which has a carboxypeptidase-inhibitory activity shows an immunopotentiating activity (J. Antibot., *37* 682, 1984).

On the basis of these findings above, new arphamenine-related compounds represented by the following general formula (I)

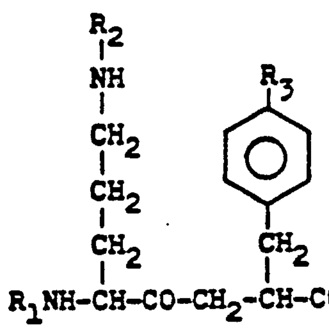

(I)

wherein $R_1$ denotes a hydrogen atom or a t-butoxycarbonyl, benzyloxycarbonyl, p-methoxy-benzyloxycarbonyl or benzoyl group as amino-protecting group, $R_2$ denotes a dimethylpyrimidinyl group, $R_3$ denotes a hydrogen atom or a hydroxyl group, and $R_4$ denotes a hydrogen atom or a methyl, ethyl, propyl, t-butyl, benzyl, p-nitrobenzyl or p-methoxybenzyl group as carboxyl-protecting group have been synthesized. These arphamenine-related compounds have been examined for their physiological activities. As a result, it has been confirmed that these compounds have a carboxy-peptidase-inhibitory activity and an immunopotentiating activity.

In general formula (I) mentioned above, $R_2$ denotes such a dimethylpyrimidinyl group derived from a guanyl group, especially 4,6-dimethylpyrimidin-2-yl group.

Examples of the medicinally acceptable or pharmaceutically acceptable salt of the compound of the general formula (I) include such salts of the carboxyl group existing in said compound with a pharmaceutically acceptable cation, for example, cations of an alkali metal such as sodium and potassium; and an alkaline earth metal such as calcium and magnesium, and ammonium ion. Examples of the salts of the compound of the general formula (1) also include such salts of the dimethylpyrimidinyl group and the amino group existing in said compound with a pharmaceutically acceptable inorganic acid or organic acid such as hydrochloric acid, hydrobromic acid, sulfuric acid, phosphoric acid, methanesulfonic acid, acetic acid, propionic acid, fumaric acid, maleic acid, malonic acid, benzoic acid, salicylic acid, phenylacetic acid and benzenesulfonic acid. The production and uses of these salts are therefore also within the scope of this invention.

Several routes for the synthesis of (2-pyrimidinylamino)-n-alkanoic acid, e.g. by cyclocondensation of 1,3-diketones with C-(guanidino)amino acids to yield (4,6-dialkyl-2-pyrimidinylamino)-n-alkanoic acids are described in Chem. Ber. *108* (1975), p. 862—874, especially page 864/5.

The arphamenine-related compound (I) according to this invention may be produced by chemical modification and salt-forming reaction of the amino group, the guanidyl group and/or the carboxyl group of arphamenine which is effected in a known manner. Thus, when the chemical modification of the amino group is to be conducted by acylation such as benzoylation, arphamenine may be reacted with benzoyl chloride in an aqueous solution of sodium carbonate. After the reaction is completed, ethyl ether is added to the reaction solution to precipitate the N-benzoyl-arphamenine. When the chemical modification of the guanidyl group of arphamenine is to be effected, for instance, by dimethylpyrimidylation, an N-protected arphamenine may be reacted with 2,4-pentanedione in a mixture of an alkali metal carbonat, for example potassium carbonate and dioxane and water at a temperature of 15 to 45°C, preferably 37°C. After the reaction is finished, the reaction mixture is adjusted to pH 5.0 to precipitate the N-protected-dimethyl-pyrimidylated arphamenine. Furthermore, when the chemical modification of the carboxyl group of arphamenine is to be effected by esterification, for example, by esterification with n-propyl, arphamenine may be kept in $BF_3$-n-propyl alcohol overnight, followed by concentrating the reaction mixture and passing the concentrated solution through a column of Sephadex LH—20, so that the arphamenine n-propyl ester is afforded. The removal of the above-defined amino-protecting groups and carboxyl-protecting groups may be achieved in a conventional manner.

The physiological activities of the compounds according to this invention are described below.

(1) Method of testing the activity of the compounds inhibitory to carboxypeptides A:

The carboxypeptidase A-inhibitory activity was determined according to a modification of the method of Hayakari et al. (M. Hayakari et al., "Analytical Biochemistry" *84*, 361—369, 1978). Thus, a mixture of 0.05 ml of a solution of 10 mM hippuryl-L-phenylalanine, 0.25 ml of 0.05 M Tris-hydrochloride buffer (pH 7.5) containing 0.9 M NaCl and 0.15 ml of a solution containing the test compound was heated at 37°C for 3 minutes. To said mixture was then added 0.05 ml of a carboxypeptidase A (Type 1, bovine pancreas, a product of Sigman Co.), and the reaction was effected at 37°C for 30 minutes. The reaction solution was admixed with 0.03 ml of aqueous 1N sodium hydroxide to stop the reaction. After allowing to stand at ambient temperature for 15 minutes, the reaction solution was admixed with 2 ml of 0.06 M phosphate buffer (pH 7.2) and with 2 ml of a solution of 1% cyanuric chloride in ethylenglycol mono-methylester. After the reaction solution was allowed to stand at ambient temperature for 5 minutes, absorbance (a) at 382 nm of the reaction solution was measured. For the blank test, the above procedure was repated using said buffer solutions without the test compound, and the absorbance (b) was measured in the blank test. The percentage of inhibition to carboxypeptidase A was calculated according to the equation:

$$\frac{(b - a)}{b} \times 100$$

On the basis of the values of inhibition percentages as measured by the above procedure, 50% Inhibition Concentration ($IC_{50}$) was evaluated. The results are shown in Table 1 below.

TABLE 1

| No. | Compounds | $IC_{50}$ (μg/ml) |
|---|---|---|
| 1 | Compound of Example 1 (b) | 0.02 |
| 2 | Compound of Example 2 (b) | 0.0088 |
| 3 | Compound of Example 3 (b) | 0.064 |
| 4 | Compound of Example 4 (b) | 0.35 |

(2) The effect on cell-mediated immunity:

The effect of the new arphamenine-related compounds on cell-mediated immunity was evaluated using as an indication a Delayed Type Hypersensitivity (D.T.H.) which is invoked in mice when sheep red blood cell as antigen was inoculated into the footpad of mice.

Test Procedure:

A suspension of $10^8$ sheep red blood cells in 0.05 ml of physiological saline was subcutaneously injected into one footpad of each $CDF_1$-mouse (female, 8 weeks-aged, 5 mice in each group). Simultaneously, the mouse under test was given orally the test compound at a dosage of 5 mg/Kg, 0.5 mg/Kg or 0.05 mg/KG as the solution which was prepared by dissolving the test compound in physiological saline and then filtering the resulting solution by a millipore filter. On 4 days after the administration of the test compound, $10^8$ sheep red blood cells were subcutaneously injected into another footpad of each test

mouse. 24 hours later, the thickness of the footpads was measured with calipers for the estimation. The test results are shown in Table 2 below.

TABLE 2

Effect on establishment of D.T.H. response with sheep red blood cells

| Test Compounds | Dosages (mg/Kg) | Degree of Swelling as compared to the control (%) |
|---|---|---|
| Compound of Example 1(b) | 5 | 128* |
| | 0.5 | 121 |
| | 0.05 | 107 |
| Compound of Example 2(b) | 5 | 144* |
| | 0.5 | 130* |
| | 0.05 | 113 |
| Compound of Example 3(b) | 5 | 136* |
| | 0.5 | 120 |
| | 0.05 | 110 |
| Compound of Example 4(b) | 5 | 145* |
| | 0.5 | 123* |
| | 0.05 | 101 |

Toxicity of the present compounds was assessed in respect of the compounds of Example 1, 2, 3 and 4 as follows: Thus, 100 mg/Kg of the test compound were intraperitoneally administered in ICR-strain mice (male, 5 weeks-aged, weight 20 g), when it was found that no death was caused by the final compounds obtained in the Examples 1 to 4.

From these observations, it is clear that the arphamenine-related compound according to this invention enhances the cell-mediated immunity at a low dosage. This strong immunopotentiating activity of the compounds of this invention shows that these compounds are useful widely in an immunotherapeutic treatment of tumors, and also as an agent of preventing infections and that these compounds are useful in the preparation of pharmaceutical compositions having immunopotentiating activity.

The arphamenine-related compounds of the formula I or their physiologically acceptable salts may be administered as active ingredients in pharaceutical compositions, i.e. in any form of oral preparations, injections and rectal suppositories when they are used as the immunopotentiating agent. These compositions may be formulated into various forms by admixing with a pharmaceutically acceptable carrier, i.e. with different kinds of pH adjustors, stabilizers, and excipients. The dosage of the arphamenine-related compounds may vary depending on conditions of the diseases, but the usual dosage may be 50 to 200 mg in oral administration for adult once a day.

This invention is now described with reference to the following Examples of this invention, to which this invention is not limited.

Example 1

(a) Synthesis of 5-benzylamino-8-guanidino-4-oxo-2-phenylmethyloctanoic acid

Arphamenine A hydrochloride (200 mg) was dissolved in 1 ml of water, and to the resulting solution were added 98 µl of benzoyl chloride and 119 mg of sodium carbonate, each in ⅕ portions at 30 minutes-interval and totally five times, under ice-cooling and stirring. After the reaction was completed, the reaction mixture was adjusted to pH 7.0 with 1N HCl, followed by addition of ethyl ether. The precipitate formed was washed with water and dried to give 225 mg of a colorless powder. This product gave a value of m/z 425 ($MH^+$) in mass spectrometry.

Elemental analysis (for $C_{23}H_{28}N_4O_4$, M.W. = 424.5):

Found: C 64.55, H 7.01, N 12.88%

Calcd.: C 65.08, H 6.65, N 13.20%

(b) Synthesis of 5-benzoylamino-8-[(4,6-dimethylpyrimidin-2-yl)amino]-4-oxo-2-phenylmethyl-octanoic acid

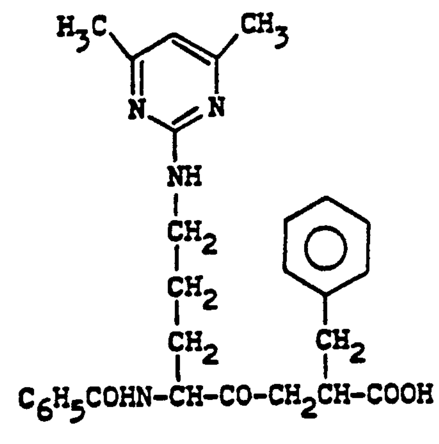

The compound (87 mg) obtained in the above step (a), acetylacetone, namely 2,4-pentanedione (126 mg) and anhydrous potassium carbonate (100 mg) were dissolved in 1.5 ml of dioxane-water (1:1), followed by stirring the solution at 37°C overnight to effect the reaction. After the reaction was completed, the reaction mixture was adjusted to pH 5.0 by addition of 1N hydrochloric acid, and concentrated to dryness. The residue obtained was chromatographed in a column of silica-gel (5 g) by eluting with a mixture of chloroform and methanol, while changing the ratio of chloroform to methanol in said mixture gradiently from 50:1 to 20:1. The reaction product was eluted out in fraction Nos. 16 to 24 [chloroform-methanol (20:1)] of the eluate. These fractions were combined together and concentrated to obtain 54 mg of a colorless powder. This product gave a value of m/z 489 ($MH^+$) in mass spectrometry.

Elemental analysis (for $C_{28}H_{32}N_4O_4$, M.W. = 488.59);

Found: C 68.66, H 6.77, N 11.27

Calcd.: C 68.83, H 6.60, N 11.47

The compound as produced in this Example 1(b) shows a melting point of 134 to 137°C, and its infrared absorption spectrum (pelleted in KBr) is as shown in Figure 1 of the attached drawings. Nuclear magnetic resonance absorption spectrum ($^1H$—NMR) of the compound of Example 1(b) in solution in deutero-methanol-deutero chloroform, (δ ppm., 200 MHz) shows absorptions at 1.52—2.03 ($CH_2 \times 2$), 2.21 ($CH_3 \times 2$), 2.39—3.29 (CH, $CH_2 \times 3$), 4.74 (CH), 6.24 (CH), 7.1—7.3 ($C_6H_5$), 7.54 ($C_6H_5$).

Example 2

(a) Synthesis of 5-benzoylamino-8-guanidino-2-(4'-hydroxyphenylmethyl)-4-oxo-octanoic acid

Arphamenine B hydrochloride (300 mg) was dissolved in 2 ml of water, and to the resulting solution were added 140 ml.of benzoyl chloride and 170 mg of sodium carbonate, each in ⅕ portions at 30 minutes-intervals and totally five times, under ice-cooling and stirring. After the reaction was completed, the reaction mixture was adjusted to pH 7.0 by addition of 1N hydrochloric acid, followed by addition of ethyl ether. The precipitate formed was washed with water and dried to give 270 mg of a colorless powder. This product gave a value of m/z 441 ($MH^+$) in mass spectrometry.

Elemental analysis (for $C_{23}H_{28}N_4O_5$, M.W. = 440.5):

Found: C 62.96, H 6.61, H 12.63

Calcd: C 62.70, H 6.41, N 12.72

(b) Synthesis of 5-benzoylamino-8-[(4,6-dimethylpyrimidin-2-yl)amino]-2-(4-hydroxyphenylmethyl)-4-oxo-octanoic acid

The compound (220 mg) obtained in the above step (a), acetylacetone, namely 2,4-pentanedion (315 mg) and anhydrous potasssium carbonate (100 mg) were dissolved in 1.5 ml of dioxane-water (1:1), and the resultant solution was stirred at 37°C overnight to effect the reaction. After the reaction was completed, the reaction mixture was adjusted to pH 5.0 with 1N hydrochloric acid. The reaction mixture was then concentrated to dryness. The residue obtained was chromatographed in a column of silica gel (5 g) by eluting with a mixture of chloroform and methanol, while changing the ratio of chloroform to methanol in said mixture gradiently from 50:1 to 10:1. The reaction product was eluted out in fractions Nos. 22 to 31 [chloroform methanol (10:1)] of the eluate. These fractions were combined together and concentrated to dryness to afford 59 mg of a colorless powder. This product gave a value of m/z 505 ($MH^+$) in mass spectrometry.

Elemental analysis (for $C_{28}H_{32}H_4O_5$, M.W. = 504.58):

Found: C 66.23, H 6.80, N 10.72

Calcd.: C 66.65, H 6.39, N 11.10

The compound as produced in this Example 2(b) shows a melting point of 110—113°C, and its infrared absorption spectrum (pelleted in KBr) is as shown in Figure 2 of the attached drawings. Nuclear magnetic resonance absorption spectrum ($^1H$—NMR) of the compound of Example 2(b) (in solution in deutero methanol-deutero chloroform (δ ppm., 200 MHz) shows absorptions at 1.26—2.19 ($CH_2 \times 2$), 2.29 ($CH_3 \times 2$), 2.29—3.30 (CH, $CH_2 \times 3$), 4.77 (CH), 6.31 (CH), 6.80 ($C_6H_4$), 7.54 ($C_6H_5$).

Example 3

(a) Synthesis of 5-benzyloxycarbonylamino-8-guanidino-4-oxo-2-phenylmethyloctanoic acid

Arphamenine A hydrochloride (150 mg), sodium hydrogen carbonate (176 mg) and S-benzyloxycarbonyl-4,6-dimethyl-2-mercaptopyrimidine (230 mg) were dissolved in 20 ml of dioxane-water (1:1), and the resultant solution was stirred at room temperature overnight. After the reaction was completed, the reaction solution was adjusted to pH 2.0 with 1N hydrochloric acid, followed by extraction with n-butanol. The n-butanol phase was washed with water and concentrated to dryness. The residue obtained was chromatographed in a column of silica-gel (20 g) by eluting with a mixture of chloroform and methanol, while changing the ratio of chloroform to methanol in said mixture gradiently from 15:1 to 2:1. The reaction product was eluted out in fraction Nos. 60 to 120 [chloroform-methanol (2:1)] of the eluate. These fractions were combined together and concentrated to dryness to afford 116 mg of a colorless powder. This product gave a value of m/z 455 ($MH^+$) in mass spectrometry.

Elemental analysis (for $C_{24}H_{30}N_4O_5 \cdot H_2O$, M.W. = 47254):

Found: C 61.37, H 6.68, N 11.70
Calcd.: C 61.0, H 6.83, N 11.86

(b) Synthesis of 5-benzylaoxycarbonylamino-8-[(4,6-dimethylpyrimidin-2-yl)amino]-4-oxo-2-phenylmethyloctanoic acid

The compound (110 mg) obtained in the above step (a), acetylacetone (150 mg) and anhydrous potassium carbonate (100 mg) were dissolved in 1.5 ml of dioxane-water (1:1), and the solution was stirred at 37°C overnight to effect the reaction. After the reaction was completed, the reaction mixture was adjusted to pH 5.7 with 1N hydrochloric acid and then concentrated to dryness. The residue obtained was chromatographed in a column of silica-gel (5 g) by eluting with a mixture of chloroform and methanol, while changing the ratio of chloroform to methanol in said mixture gradiently from 50:1 to 10:1. The reaction product was eluted out in fraction Nos. 8 to 70 of the eluate. These fractions were combined together and concentrated to dryness to afford 78 mg of a colorless powder. This product gave a value of m/z 519 ($MH^+$) in mass spectrometry.

The compound as produced in this Example 3(b) has an infrared absorption spectrum (pelleted in KBr) as shown in Figure 3 of the attached drawings. Nuclear magnetic resonance absorption spectrum ($^1H$—NMR) of the compound of Example 3(b) (in solution in deutero chloroform, δ ppm., 200 MHz) shows absorptions at 1.48—2.00 ($CH_2 \times 2$), 2.23 ($CH_3 \times 2$), 2.45—3.45 (CH, $CH_2 \times 3$), 4.26 (CH), 5.00 (CH), 6.16 ($C_6H_4$, 7.05—727 ($C_6H_5 \times 2$;.

Elemental analysis (for $C_{29}H_{34}N_4O_5 \cdot \frac{1}{2}H_2O$, M.W. = 527.62):

Found: C 66.39, H 6.70, N 10.52
Calcd.: C 66.02, H 6.69, N 10.62

Example 4

(a) Synthesis of 5-benzyloxycarbonylamino-8-guanidino-2-(4′-hydroxyphenylmethyl)-4-oxo-octanoic acid

Arphamenine B hydrochloride (150 mg), sodium hydrogen carbonate (176 mg) and carbobenzoxy S-4,6-dimethylpyrimidin-2-ylthiolcarbonate (230 mg) were dissolved in 20 ml of dioxane-water (1:1), and the solution was stirred at room temperature overnight. After the reaction was completed, the reaction solution was adjusted to pH 2.0 with 1N hydrochloric acid, followed by extraction with n-butanol. The n-butanol phase was washed with water and then concentrated to dryness. The residue obtained was dissolved in water containing 40% methanol, and the resulting solution was passed through a column of CM-Sephadex C—25 ($H^+$, 15 ml). The column was washed with 40% methanol-water and then eluted with water containing 40% methanol and 0.01N hydrochloric acid. The reaction product was eluted out in fraction Nos. 8 to 20 of the eluate. These fractions were combined together and adjusted to pH 2.0, and again extracted with n-butanol. The n-butanol phase obtained was washed with water and then concentrated to dryness to obtain 167 mg of a colorless powder. This product gave a value of m/z 471 ($MH^+$) in mass spectrometry.

Elemental analysis (for $C_{24}H_{30}N_4O_6 \cdot \frac{1}{2}H_2O$, M.W. = 479.53):

Found: C 60.41, H 6.55, N 11.51
Calcd.: C 60.11, H 6.52, N 11.68

(b) Synthesis of 5-benzyloxycarbonylamino-8-[(4,6-dimethylpyrimidin-2-yl)amino]-2-(4′-hydroxyphenylmethyl)-4-oxo-octanoic acid

The compound (150 mg) obtained in the above step (a), acetylacetone (200 mg) and anhydrous potassium carbonate (100 mg) were dissolved in 1.5 ml of dioxane-water (1:1), and the solution was stirred at 37°C overnight to effect the reaction. After the reaction was completed, the reaction solution was adjusted to pH 5.7 with 1N hydrochloric acid and concentrated to dryness. The residue obtained was

dissolved in methanol and the resultant solution was filtered to remove methanol-insoluble matters therefrom. The solution as filtered was chromatographed in a column of silica-gel (5 g) by eluting with a mixture of chloroform and methanol, while changing the ratio of chloroform to methanol in said mixture gradiently from 50:1 to 10:1. The reaction product was eluted out in fraction Nos. 13 to 41 of the eluate. These fractions were combined together and concentrated to dryness to afford 109 mg of a colorless powder. This product gave a value of m/z 544 ($MH^+$) in mass spectrometry.

Elemental analysis (for $C_{29}H_{34}N_4O_6 \cdot \frac{1}{2}H_2O$, M.W. = 543.62):

Found: C 64.13, H 6.26, N 9.96

Calcd.: C 64.07, H 6.49, N 10.31

The compound as produced in this Example 4(b) shows a melting point of 100—103°C, and its infrared absorption spectrum (pelleted in KBr) is as shown in Figure 4 of the attached drawings. Nuclear magnetic resonance absorption spectrum ($^1H$—NMR) of the compound of Example 4(b) (in solution in deutero chloroform, δ ppm., 200 MHz) shows absorptions at 1.47—1.98 ($CH_2 \times 2$), 2.26 ($CH_3 \times 2$), 2.35—3.44 (CH, $CH_2 \times 3$), 4.14 (CH), 5.06 ($CH_2$), 6.35 (CH), 6.83 ($C_6H_4$), 7.26—7.38 ($C_6H_5$).

4. Brief Explanation of Drawings

Figure 1, Figure 2, Figure 3 and Figure 4 show the infrared absorption spectra of the compounds of this invention as produced in Example 1(b), Example 2(b), Example 3(b) and Example 4(b) according to this invention, respectively.

**Claims for the Contracting States: BE CH DE FR GB IT LI LU NL SE**

1. An arphamenine-related compound represented by the general formula:

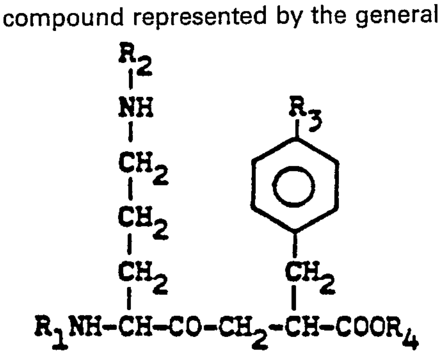

(I)

wherein $R_1$ denotes a hydrogen atom or a t-butoxycarbonyl, benzyloxycarbonal, p-methoxybenzyloxycarbonyl or benzoyl group as amino-protecting group, $R_2$ denotes a dimethylpyrimidinyl group, $R_3$ denotes a hydrogen atom or a hydroxy group, and $R_4$ denotes a hydrogen atom or a methyl, ethyl, propyl, t-butyl, benzyl, p-nitrobenzyl or p-methoxybenzyl group as carboxyl-protecting group, and a pharmaceutically acceptable salt thereof.

2. A compound as claimed in claim 1, in which $R_1$ is a benzoyl group or a benzyloxycarbonyl group, $R_2$ is a dimethylpyrimidinyl group, $R_3$ is a hydrogen atom or a hydroxyl group, and $R_4$ is a hydrogen atom in the general formula (I).

3. A process for the preparation of a compound of the formula I

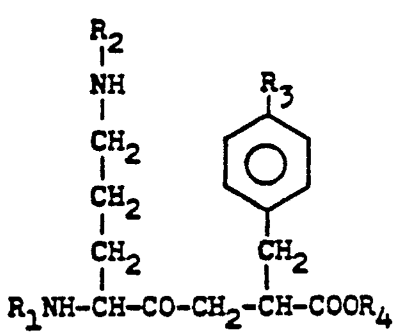

(I)

wherein $R_1$, $R_2$, $R_3$ and $R_4$ are as defined in claim 1, or a pharmaceutically acceptable salt thereof, which comprises reacting an N-protected arphamenine of the formula II

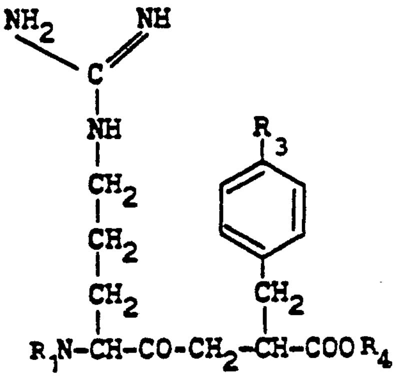

(II)

wherein $R_3$ and $R_4$ are as defined in claim 1 and $R_1$ is one of the in claim 1 defined amino-protecting groups, with 2,4-pentanedione, and optionally removing the in claim 1 defined amino-protecting and carboxyl-protecting groups in a conventional manner.

4. The process as claimed in claim 2 wherein the reaction with 2,4-pentanedione is carried through in a mixture of an alkali metal carbonate, dioxane and water at a temperature of from 15 to 45°C.

5. The process as claimed in claim 3 wherein a mixture of potassium carbonate, dioxane and water is used at a temperature of 37°C.

6. A pharmaceutical composition comprising a compound of the formula I as claimed in claim 1, or a physiologically acceptable salt thereof, as the active ingredient, and a pharmaceutically acceptable carrier.

7. Use of a compound as claimed in claim 1 for the preparation of a pharmaceutical composition having immuno-potentiating activity.

**Claims for the Contracting State: AT**

1. A process for the preparation of a compound of the formula I

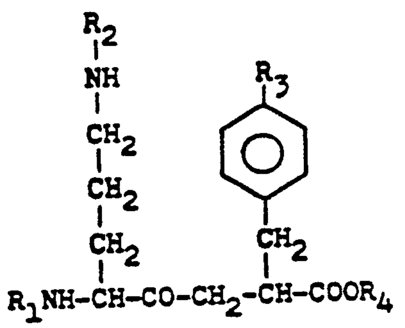

(I)

wherein $R_1$ denotes a hydrogen atom or a t-butoxycarbonyl, benzyloxycarbonyl, p-methoxy-benzyloxycarbonyl or benzoyl group as amino-protecting group, $R_2$ denotes a dimethylpyrimidinyl group, $R_3$ denotes a hydrogen atom or a hydroxyl group, and $R_4$ denoted a hydrogen atom or a methyl, ethyl, propyl, t-butyl, benzyl, p-nitrobenzyl or p-methoxybenzyl group as carboxyl-protecting group, or a pharmaceutically acceptable salt thereof, which comprises reacting an N-protected arphamenine of the formula II

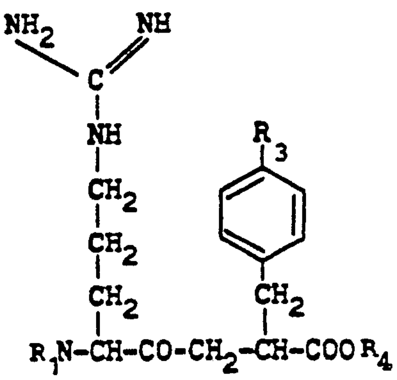

(II)

wherein $R_3$ and $R_4$ are as defined above and $R_1$ is one of the above defined amino-protecting groups, with 2,4-pentanedione, and optionally removing the above-defined amino-protecting and carboxyl-protecting groups in a conventional manner.

2. The process as claimed in claim 1 wherein the reaction with 2,4-pentanedione is carried through in a mixture of an alkali metal carbonate, dioxane and water at a temperature of from 15 to 45°C.

3. The process as claimed in claim 2 wherein a mixture of potassium carbonate, dioxane and water is used at a temperature of 37°C.

4. The process as claimed in claim 1, in which $R_1$ is a benzoyl group or a benzyloxycarbonyl group, $R_2$ is a dimethylpyrimidinyl group, $R_3$ is a hydrogen atom or a hydroxyl group, and $R_4$ is a hydrogen atom in the general formula (I).

**Patentansprüche für die Vertragsstaaten: BE CH DE FR GB IT LI LU NL SE**

1. Eine mit Arphamenin verwandte Verbindung, die durch die folgende allgemeine Formel dargestellt wird:

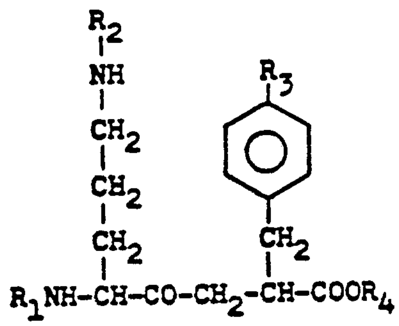

(I)

worin $R_1$ ein Wasserstofatom oder eine t-Butoxycarbonyl-, Benzyloxycarbonyl-, p-Methoxy-benzyloxycarbonyl- oder Benzoyl- gruppe als Aminoschutzgruppe darstellt, $R_2$ eine Methylpyrimidinylgruppe, $R_3$ ein Wasserstoffatom oder eine Hydroxylgruppe und $R_4$ eine Wasserstoffatom oder eine Methyl-, Ethyl-, Propyl-, t-Butyl-, Benzyl-, p-Nitrobenzyl- oder p-Methoxybenzylgruppe als Carboxylschutzgruppe bedeutet, und eines ihrer pharmaceutisch unbedenklichen Salze.

2. Eine wie in Anspruch 1 beanspruchte Verbindung, bei der in der allgemeinen Formel (I) $R_1$ eine Benzoylgruppe oder eine Benzyloxycarbonylgruppe, $R_2$ eine Dimethylpyrimidinylgruppe, $R_3$ ein Wasserstoffatom oder eine Hydroxylgruppe und $R_4$ ein Wasserstoffatom bedeutet.

3. Ein Verfahren zur Herstellung einer Verbindung der Formel I

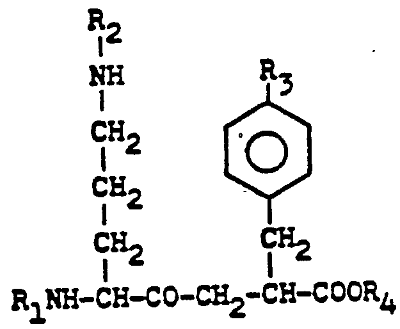

(I)

worin $R_1$, $R_2$, $R_3$ und $R_4$ wie in Anspruch 1 definiert sind, oder eines ihrer pharmaceutisch unbedenklichen Salze, gekennzeichnet durch das Umsetzen eines N-geschützten Arphamenins der Formel II

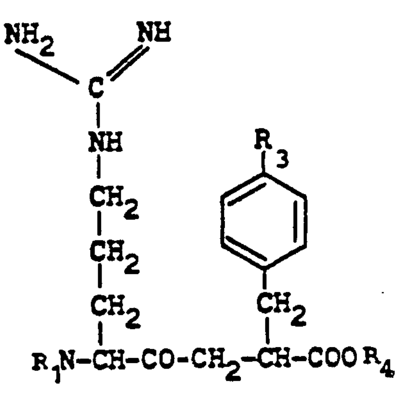

(II)

worin $R_3$ und $R_4$ wie in Anspruch 1 definiert sind und $R_1$ eine der in Anspruch 1 definierten Aminoschutzgruppen bedeutet, mit 2,4-Pentandion und das wahlweise Entfernen der in Anspruch 1 definierten Amino- und Carboxylschutzgruppen auf herkömmliche Art und Weise.

4. Das wie in Anspruch 3 beanspruchte Verfahren, worin die Umsetzung mit 2,4-Pentandion in einer Mischung aus einem Alkalimetallcarbonat, Dioxan und Wasser bei einer Temperatur von 15 bis 45°C durchgeführt wird.

5. Das wie in Anspruch 4 beanspruchte Verfahren, bei dem eine Mischung von Kaliumcarbonat, Dioxan und Wasser bei einer Temperatur von 37°C verwendet wird.

6. Eine pharmazeutische Zusammensetzung, gekennzeichnet durch eine Verbindung der Formel I wie in Anspruch 1 beansprucht oder eines ihrer physiologisch unbedenklichen Salze als Wirkstoff und einen pharmazeutisch unbedenklichen Träger.

7. Die Verwendung einer wie in Anspruch 1 beanspruchten Verbindung zur Herstellung einer pharmazeutischen Zusammensetzung mit immunitätsverstärkender Wirkung.

**Patentansprüche für den Vertragsstaat: AT**

1. Ein Verfahren zur Herstellung einer Verbindung der Formel I

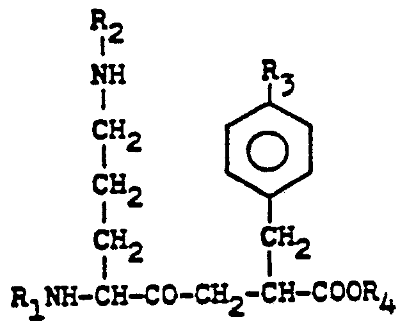

(I)

worin $R_1$ eine Wasserstoffatom oder eine t-Butoxycarbonyl-, Benzyloxycarbonyl-, p-Methoxy-benzyloxycarbonyl- oder Benzoylgruppe als Aminoschutzgruppe darstellt, $R_2$ eine Methylpyrimidinylgruppe, $R_3$ ein Wasserstoffatom oder eine Hydroxylgruppe und $R_4$ ein Wasserstoffatom oder eine Methyl-, Ethyl-, Propyl-, t-Butyl-, Benzyl-, p-Nitrobenzyl- oder p-Methoxybenzylgruppe als Carboxylschutzgruppe bedeutet, oder eines ihrer pharmazeutisch unbedenklicken Salze, gekennzeichnet durch das Umsetzen eines N-geschützten Arphamenins der Formel II

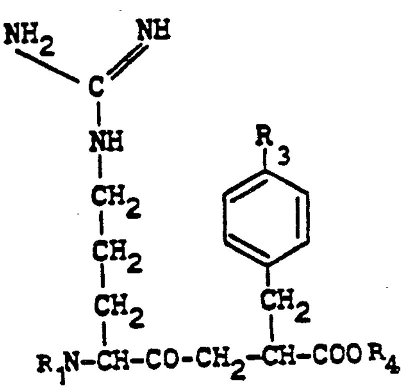

(II)

worin $R_3$ und $R_4$ wie oben definiert sind und $R_1$ eine der oben definierten Aminoschutzgruppen bedeutet, mit 2,4-Pentandion und das wahlweise Entfernen der oben definierten Amino- und Carboxylschutzgruppen auf herkömmliche Art und Weise.

2. Das wie in Anspruch 1 beanspruchte Verfahren, worin die Umsetzung mit 2,4-Pantandion in einer Mischung aus einem Alkalimetallcarbonat, Dioxan und Wasser bei einer Temperature von 15 bis 45°C durchgeführt wird.

3. Das wie in Anspruch 2 beanspruchte Verfahren, bei dem eine Mischung von Kaliumcarbonat, Dioxan und Wasser bei einer Temperatur von 37°C verwendet wird.

4. Das wie in Anspruch 1 beanspruchte Verfahren, bei dem in der allgemeinen Formel (I) $R_1$ eine Benzoylgruppe oder eine Benzyloxycarbonylgruppe, $R_2$ eine Dimethylpyrimidinylgruppe, $R_3$ ein Wasserstoffatom oder ein Hydroxylgruppe und $R_4$ ein Wasserstoffatom bedeutet.

**Revendications pour les Etats contractants: BE CH DE FR GB IT LI LU NL SE**

1. Composé dérivé d'arphaménine correspondant à la formule générale:

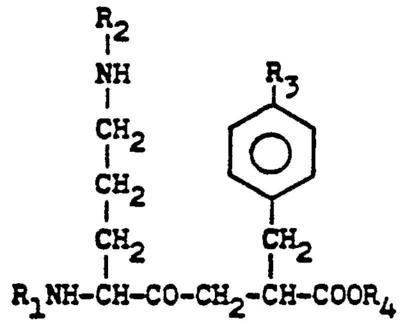

(I)

dans laquelle $R_1$ représente un atome d'hydrogène ou un groupe amino-protecteur choisi parmi les groupes tert-butoxycarbonyle, benzyloxycarbonyle, p-méthoxy-benzyloxycarbonyle et benzoyle; $R_2$ représente un groupe diméthylpyrimidinyle; $R_3$ représente un atome d'hydrogène ou un groupe hydroxyle; et $R_4$ représente un atome d'hydrogène ou un groupe carboxy-protecteur choisi parmi les groupes méthyle, éthyle, propyle, tert-butyle, benzyle, p-nitrobenzyle et p-méthoxybenzyle; ainsi qu'un sel pharmaceutiquement acceptable de celui-ci.

2. Composé selon la revendication 1, dans lequel $R_1$ représente un groupe benzoyle ou un groupe benzyloxycarbonyle, $R_2$ représente un groupe diméthylpyrimidinyle, $R_3$ représente un atome d'hydrogène ou un groupe hydroxyle, et $R_4$ représente un atome d'hydrogène dans la formule générale (I).

3. Procédé de préparation d'un composé de formule (I):

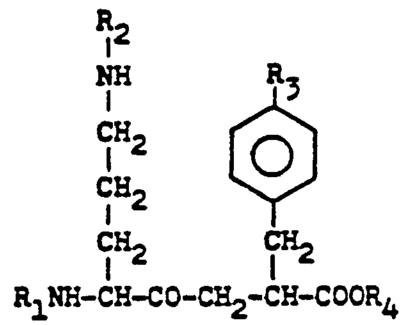

(I)

dans laquelle les groupes $R_1$, $R_2$, $R_3$ et $R_4$ sont tels que définis dans la revendication 1, ou un sel pharmaceutiquement acceptable de celui-ci, dans lequel on fait réagir une arphaménine N-protégée de formule (II):

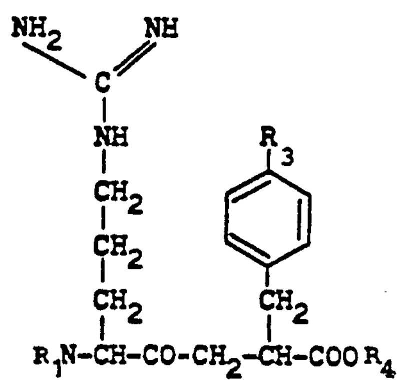

(II)

dans laquelle les groupes $R_3$ et $R_4$ sont tels que définis dans la revendication 1, et le groupe $R_1$ représente l'un des groupes amino-protecteurs définis dans la revendication 1, avec de la 2,4-pentanedione, et on élimine éventuellement de façon classique les groupes amino-protecteurs et carboxy-protecteurs définis dans la revendication 1.

4. Procédé selon la revendication 2, dans lequel la réaction avec la 2,4-pentanedione est effectuée dans un mélange de'un carbonate de métal alcalin, de dioxanne et d'eau à une température de 15 à 45°C.

5. Procédé selon la revendication 3, dans laquel on utilise un mélange de carbonate de potassium, de dioxanne et d'eau à une température de 37°C.

6. Composition pharmaceutique, comprenant un composé de formule I selon la revendication 1, ou un sel physiologiquement acceptable de celui-ci, comme ingrédient actif, ainsi qu'un véhicule pharmaceutiquement acceptable.

7. Utilisation d'un composé selon la revendication 1 pour la préparation d'une composition pharmaceutique ayant une activité immunopotentiatrice.

**Revendications pour l'Etat contractant: AT**

1. Procédé de préparation d'un composé de formule (I):

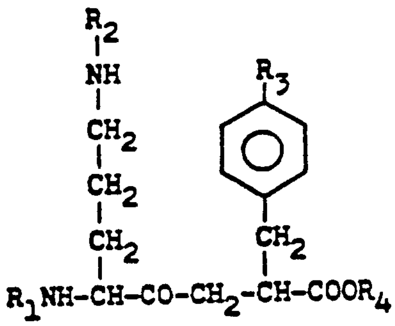

(I)

dans laquelle $R_1$ représente un atome d'hydrogène ou un groupe amino-protecteur choisi parmi les groupes tert-butoxycarbonyle, benzyloxycarbonyle, p-méthoxy-benzyloxycarbonyle, et benzoyle; $R_2$ représente un groupe diméthylpyrimidinyle; $R_3$ représente un atome d'hydrogène ou un groupe hydroxyle; et $R_4$ représente un atome d'hydrogène ou un groupe carboxy-protecteur choisi parmi les groupes méthyle, éthyle, propyle, tert-butyle, benzyle, p-nitrobenzyle et p-méthoxybenzyle; ou un sel pharmaceutiquement acceptable de celui-ci, dans lequel on fait réagir une arphaménine N-protégée de formule (II):

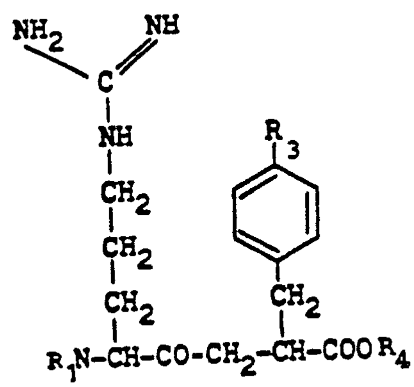

(II)

dans laquelle les groupes $R_3$ et $R_4$ sont tels que définis ci-dessus et $R_1$ représente l'un des groupes amino-protecteurs définis ci-dessus, avec de la 2,4-pentandione, et on élimine éventuellement de façon classique les gorupes amino-protecteurs et carboxy-protecteurs définis ci-dessus.

2. Procédé selon la revendication 1, dans lequel la réaction avec la 2,4-pentanedione est effectuée dans un mélange d'un carbonate de métal alcalin, de dioxanne et d'eau à une température de 15 à 45°C.

3. Procédé selon la revendication 2, dans lequel on utilise un mélange de carbonate de potassium, de dioxanne et d'eau à une température de 37°C.

4. Procédé selon la revendication 1, dans lequel $R_1$ représente un groupe benzoyle ou un groupe benzyloxycarbonyle, $R_2$ représente un groupe diméthylpyrimidinyle, $R_3$ représente un atome d'hydrogène ou un groupe hydroxyle, et $R_4$ représente un atome d'hydrogène dans la formule générale (I).

FIG.1

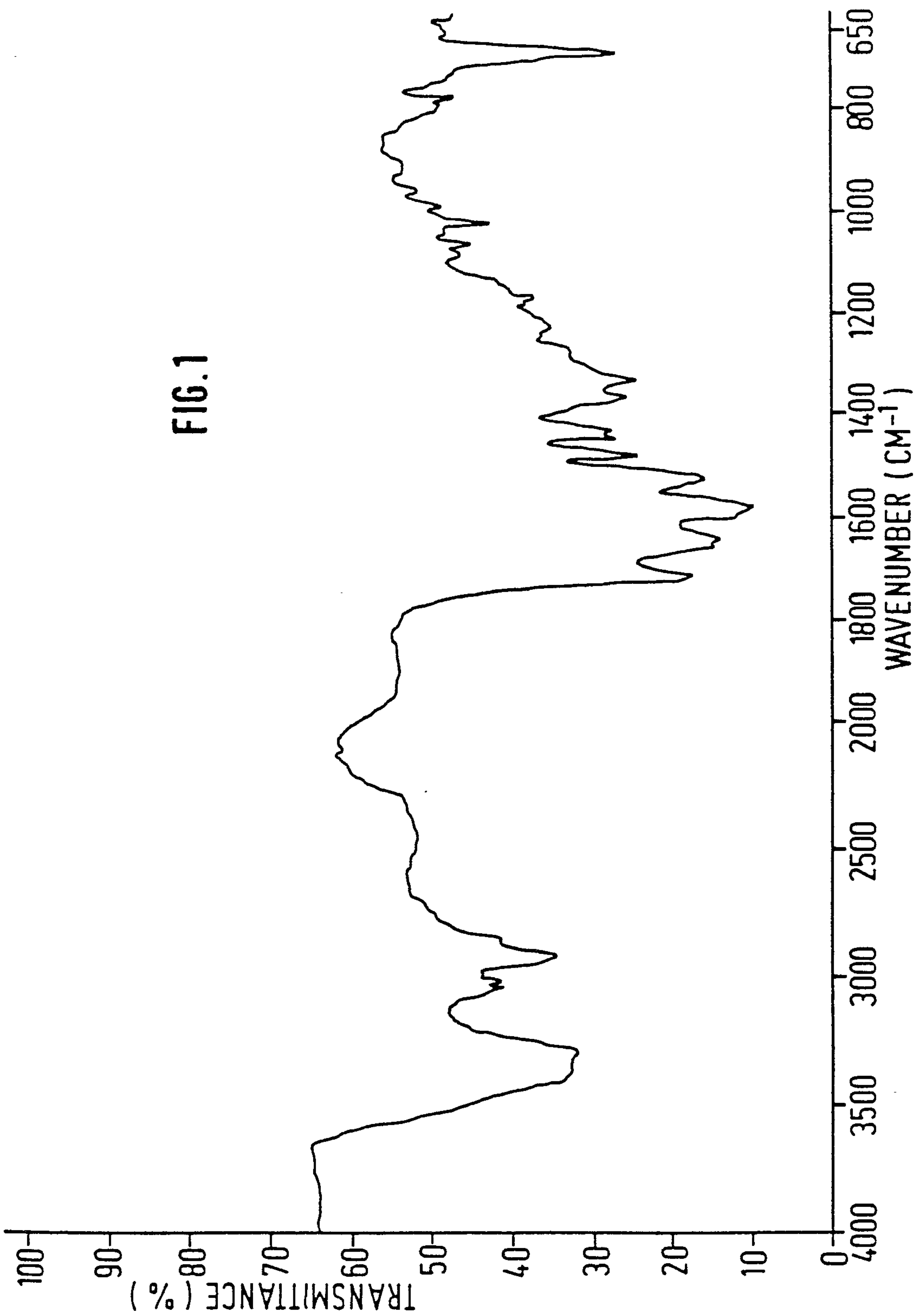
TRANSMITTANCE ( % )
0
10
20
30
40
50
60
70
80
90
100
4000
3500
3000
2500
2000
1800
1600
1400
1200
1000
800
650
WAVENUMBER ( CM$^{-1}$ )

FIG. 2

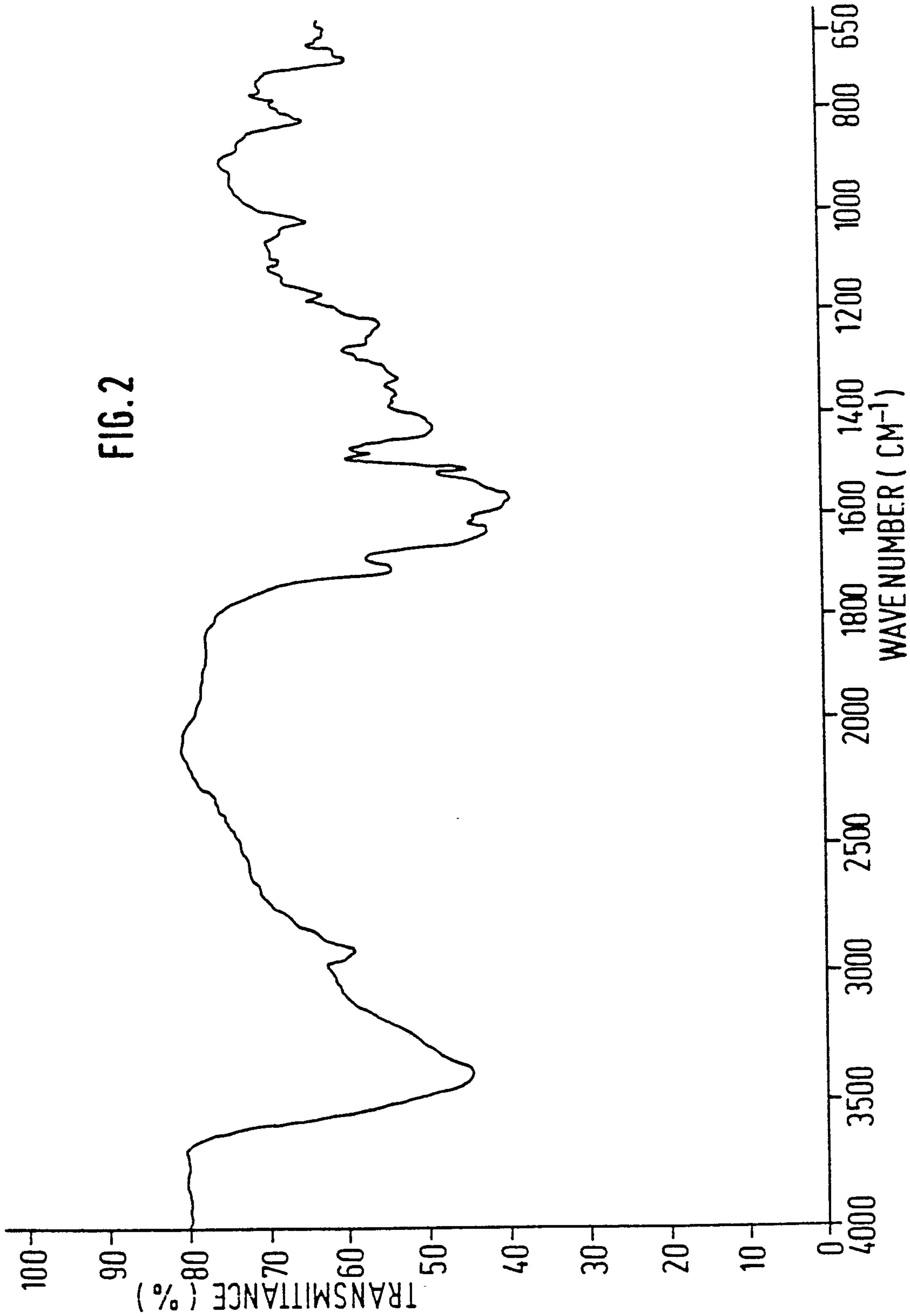
TRANSMITTANCE ( % )
100
90
80
70
60
50
40
30
20
10
0
4000
3500
3000
2500
2000
1800
1600
1400
1200
1000
800
650
WAVENUMBER ( CM⁻¹ )

FIG. 3

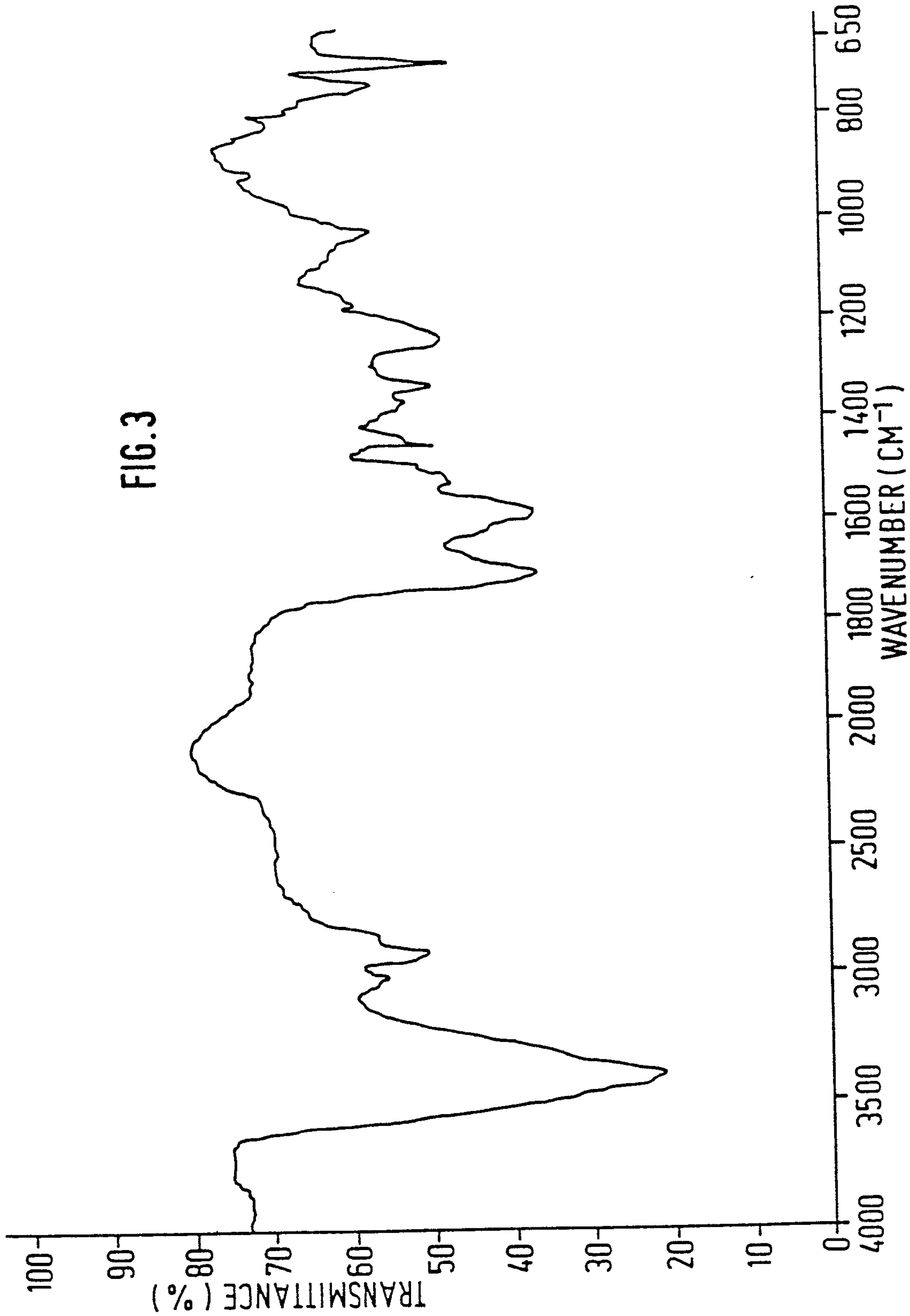
TRANSMITTANCE ( % )
100
90
80
70
60
50
40
30
20
10
0
WAVENUMBER (CM$^{-1}$)
4000
3500
3000
2500
2000
1800
1600
1400
1200
1000
800
650

FIG. 4

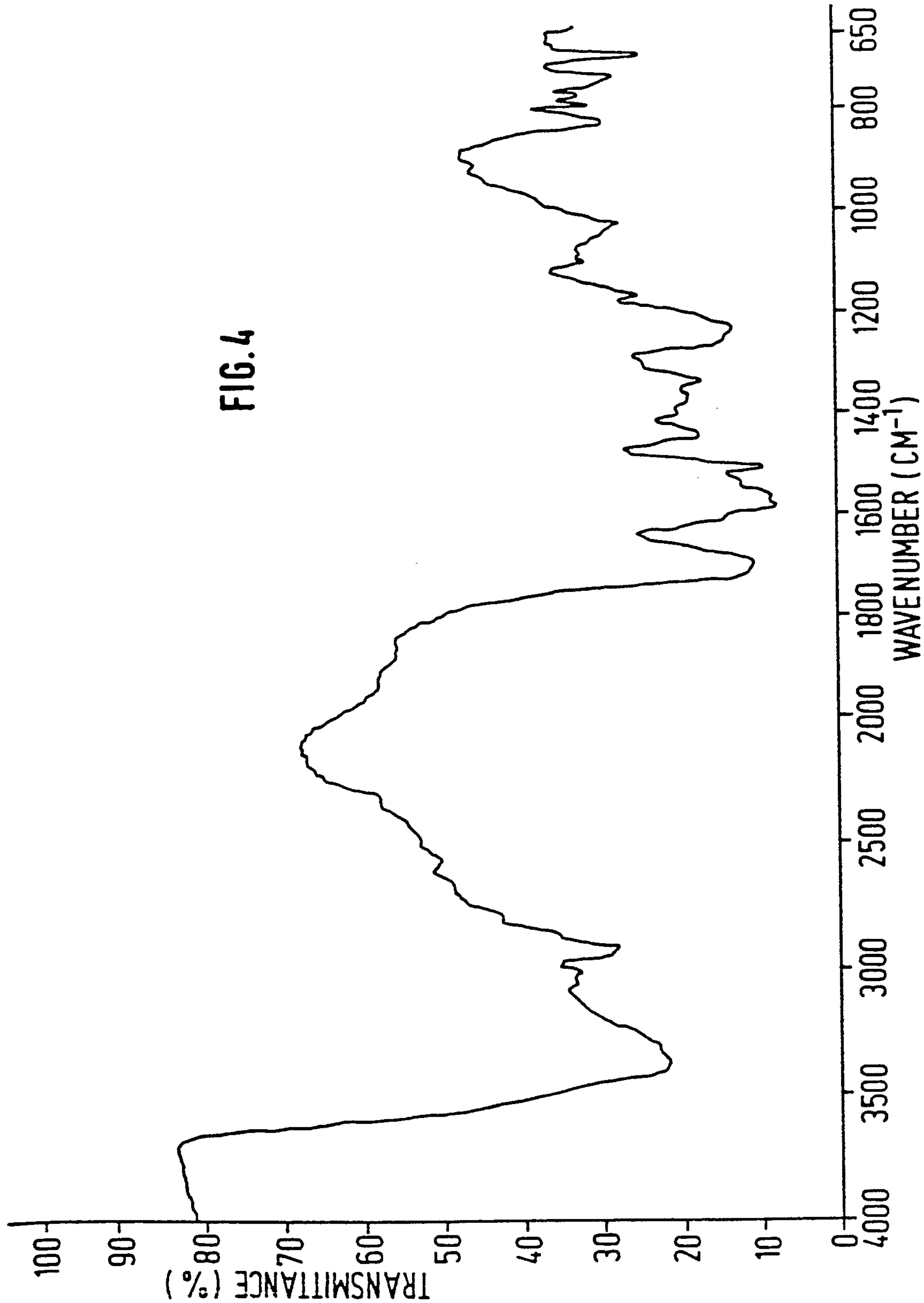
TRANSMITTANCE (%)
100
90
80
70
60
50
40
30
20
10
0
4000
3500
3000
2500
2000
1800
1600
1400
1200
1000
800
650
WAVENUMBER (CM$^{-1}$)